# EUROPEAN PATENT APPLICATION

(11) **EP 4 764 116 A1**
(43) Date of publication of application: **24.06.2026**
(21) Application number: 25225540.1
(22) Date of filing: 19.12.2025
(51) Int. Cl.: E04H 3/08, A61G 3/00, A61G 10/00, A61N 5/10, E04B 1/343

(54) **TRANSPORTABLE MEDICAL UNIT FOR RADIOLIGAND THERAPY TREATMENT**

(30) Priority: 19.12.2024 GB 202418647
(71) Applicant: InHealth Ltd, Beaconsfield HP9 2JH (GB)
(72) Inventor: TOOP, Ralph, Beaconsfield HP9 2JH (GB); BIRD, Simon, Beaconsfield HP9 2JH (GB)
(74) Representative: Page White Farrer

(57) **Abstract**

There is disclosed a transportable medical unit for the provision of radioligand therapy or molecular radiotherapy services, comprising: one or more treatment rooms, each treatment room having lead walls and a lead ceiling, wherein in a first state, the unit is lifted off the ground, and in a second state the unit is placed on the ground such that the floor of the unit is in contact with the ground.

## Description

### Field

The present disclosure relates to a transportable medical unit. More particularly, the present disclosure relates to a transportable medical unit in which a radioligand therapy or molecular radiotherapy medical procedure can be carried out.

### Background

Transportable or mobile medical units may be transported by a vehicle, and moved from one site to another in order to provide a medical service at multiple locations over time.

Such mobile medical units enable a service to be taken to patients, which may increase uptake of medical procedures or at least make having a medical procedure more convenient for a patient, and allow a service to be provided to capacity restrained sites.

### Summary

There is disclosed a transportable medical unit for the provision of radioligand therapy or molecular radiotherapy services, comprising: one or more treatment rooms, each treatment room having lead walls and a lead ceiling, wherein in a first state, the unit is lifted off the ground, and in a second state the unit is placed on the ground such that the floor of the unit is in contact with the ground.

In the first state, the unit may be transported. In the second state, the unit may be deployed for use.

The medical unit may be further configured to be transformable from a first configuration in which the medical unit is in a non-expanded state and a second configuration in which the medical unit is in an expanded state, the transportable medical unit comprising: a first portion which is configured to remain static whilst the medical unit is transformed from the first configuration to the second configuration, and a second portion which is configured to move outwardly when the medical unit is transformed from its first configuration to its second configuration.

The transportable medical unit may further comprise one or more treatment rooms. Each of the one or more treatment rooms may be configured for radioligand therapy or molecular radiotherapy treatment, and comprises a lead encapsulated treatment room. A lead encapsulated treatment room may include one or more of lead lined walls, doors, ceilings and windows, to reduce and contain radioactive exposure to patients, staff and general public from radioligand therapy or molecular radiotherapy treatments/administrations. Each of the one or more treatment rooms may be configured for radioligand therapy or molecular radiotherapy treatment, and includes a lead ceiling, doors, windows and walls.

Any join between panels forming any one of the lead lined walls, doors, ceilings and windows may be sealed with sealing components.

The lead encapsulated treatment room may not comprise a lead floor, and in the second state the location of the floor of the medical unit on the ground provides a radioligand therapy or molecular radiotherapy sealing that a lead floor would otherwise provide. The transportable medical unit of any one of claims 5 to 10 when dependent on claim 4, wherein the one or more treatment units are provided in the first portion.

Any adaptations or modifications associated with radioligand therapy or molecular radiotherapy treatment, including lead wall where provided, may be provided in the first portion.

The second portion may provide a corridor to connect the rooms of the first portion.

The floor of the first portion may be sealed to prevent ingress of contaminants into the first portion.

The transportable medical unit may further comprise hydraulic legs. In the first state the hydraulic legs may be retracted at least partially into the transportable medical unit, and in the second state the hydraulic legs may be extended from the transportable medical unit to engage with the ground.

The transportable medical unit may further comprise wheels. The transportable medical may further comprise a hot lab. The transportable medical unit may further comprise an observation room.

The transportable medical unit may further comprise a toilet. The toilet may be configured to separately handle radioactive human waste. The toilet may be configured to convert aqueous radioactive waste into solid radioactive waste. Less than 1% of the radioactive waste created may go into the environmental via waste water systems, with 99% being captured and converted into solid radioactive waste. The solid radioactive waste may be stored until decayed and a reading is at background radiation levels, then disposed/incinerated of via normal clinical waste.

The transportable medical may comprise four treatment rooms.

### Brief description of drawings

The invention is described by way of examples with reference to the accompanying drawings, in which:
FIGS. 1(a), 1(c) and 1(e) show end views of an exemplary transportable medical unit in respective transport, deployment and use states, and FIGS. 1(b), 1(d) and 1(f) show corresponding side views of the exemplary transportable medical unit in respective transport, deployment and use states;
FIGS. 2(a) and 2(b) show end views of the exemplary transportable medical unit, in a use state, when retracted and extended respectively; and
FIG. 3 is a plan view of an example layout of the exemplary transportable medical unit.

### Detailed description

Challenges arise in providing mobile or transportable medical units for radioligand therapy medical procedures. Radioligand therapy is used in the treatment of certain types of cancer, and is expected to play a much more integral role in cancer treatment in the near future. Radioligand Therapy (RLT) is sometimes referred to as Molecular Radiotherapy (MRT).

A characteristic of radioligand therapy medical procedures is that patients are injected with a radioactive isotope, together using a specific molecule(s) or ligand(s), which are a targeting compound that directs the radioactive isotope to the targeted cancer cells. Due to the use of radioactive isotopes, radioligand therapy necessitates the requirement for safety in the environment in which the procedures are conducted. Exposure to radiation must be minimised between patients, for medical staff, and for the general public, which in turn requires minimising radiation exposure to the surrounding environment.

A typical environment for providing radioligand therapy medical procedures requires constructing a treatment room(s) and laboratory with lead walls, ceilings and floorings in order to provide the necessary safety procedures. In a medical procedure environment, such as a hospital or clinic, this creates challenges in respect of the extra space required and - significantly - the extra structural weight and cost needed.

One of the advantages of mobile medical treatment units is that they can be moved between sites, so that rather than a single clinic or hospital having to find the space for the unit or committing to the cost for a unit, space can be saved and costs reduced by providing the medical treatment unit temporarily.

For radioligand therapy medical treatments an additional problem arises due to the need to fulfil the necessary safety procedures, and specifically to provide the unit with lead walls, ceilings, and floors. The associated weight involved in providing a mobile or transportable medical unit for radioligand therapy treatment, makes providing such a unit challenging.

It is also desirable for a treatment unit to be able to provide multiple treatment bays, to allow treatment to be provided to multiple patients at one time. This presents a further challenge of space and cost in a static environment, and it is desirable for a mobile unit to have the provision for treating multiple patients at one time, by having multiple bays.

This creates an additional challenge for a mobile radioligand therapy medical treatment unit. If multiple bays are provided, then the safety provisions need to be provided for each bay. This increases the requirements for lead in the unit, increasing the challenge in providing a mobile radioligand therapy medical treatment unit.

It is desirable to provide a mobile radioligand therapy medical treatment unit, which allows safety provisions to be met, and which allows treatment of multiple patients simultaneously. It is desirable to achieve this in a single mobile unit, preferably with all aspects associated with the provision of radioligand therapy treatment contained within a single mobile unit.

The invention addresses the technical challenge of providing a radioligand therapy treatment unit having the necessary safety requirements within the compact dimensions of a mobile unit, and preferably providing for the treatment of multiple patients simultaneously.

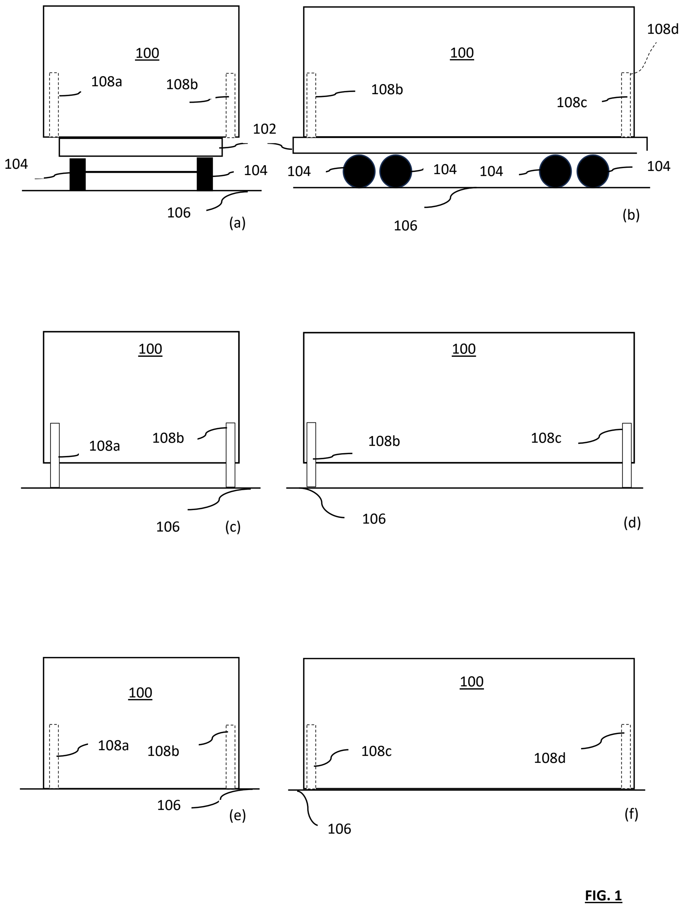

With reference to FIGS. 1(a) to 1(f), there is illustrated a transportable (or mobile) medical treatment radioligand therapy unit 100. The transportable (or mobile) medical treatment radioligand therapy unit 100 is referred to as treatment unit 100.

FIGS. 1(a) and 1(b) respectively show end and side views of the transportable treatment unit 100 in a first stage of operation, which may be termed a transport stage.

The treatment unit 100 is positioned on a trailer 102. The trailer 102 may be a trailer towed by a vehicle, or may be a flatbed of a vehicle rather than a towed trailer. The term 'trailer' is used to encompass any means for transporting the transportable treatment unit 100 between locations. The trailer 102 is shown as having wheels 104, and moves along the ground 106.

The transportable treatment unit 100 is provided with four hydraulic legs 108a, 108b, 108c, 108d, preferably positioned in each of the four corners of the treatment unit 100. In the transport stage, the hydraulic legs are withdrawn into the body of the treatment unit, and hence in FIGS. 1(a) and 1(b) they are illustrated in dashed lines, as they are not externally visible. A part of each hydraulic leg may extend beneath the treatment unit 100. In practice, the hydraulic legs may not be withdrawn fully into the body of the treatment unit 100, but may extend partially from the body in the transport stage. Hydraulic leg 108d is not illustrated in FIGS. 1(a) and 1(b), as it is in the corner which is not at the end or side of FIGS. 1(a) and 1(b). In FIG. 1(b) a dashed line to reference numeral 108d, points to a hydraulic leg which is behind the hydraulic leg 108c (and similarly hydraulic leg 108d would be behind hydraulic leg 108a in FIG. 1(a)).

FIGS. 1(c) and 1(d) respectively show end and side views of the transportable treatment unit 100 in a second stage of operation, which may be termed a deployment stage.

The hydraulic legs 108a, 108b, 108c, 108d are deployed, to extend from the body of the transportable treatment unit 100 to contact the ground 106, and then to continue extending in order to raise the base of the transportable treatment unit 100 off the trailer 102 surface. With the transportable treatment unit 100 raised from the trailer surface, the trailer can be removed (e.g. towed away), leaving the medical unit supported above the ground by the four hydraulic legs, as shown in FIGS. 1(c) and 1(d).

FIGS. 1(e) and 1(f) respectively show end and side views of the transportable treatment unit 100 in a third stage of operation, which may be termed a use stage.

The hydraulic legs 108a, 108b, 108c, 108d are withdrawn into the body of the transportable treatment unit 100, to lower the transportable treatment unit 100 toward the ground 106, such that the base of floor of the transportable treatment unit 100 sits on and is in contact with the ground 106.

Advantageously, by positioning the transportable unit 100 on the ground for use, such that it's floor is in direct contact with the ground, there is no requirement to provide a lead barrier on the floor of the transportable unit. The weight associated with providing a lead floor is thus saved.

Advantageously, by positioning the transportable unit 100 on the ground for use, such that it's floor is in contact with the ground, there is enhanced accessibility for patients and staff, with no requirement for a lift or stairs as there is a ramp for access on/off the unit.

Alternative techniques may be provided for transporting and deploying the transportable unit. The unit 100 may itself be provided with wheels, and may itself be a trailer. Where provided with wheels, a mechanism is provided to allow the transportable unit to be deployed for use with its bottom surface engaged with the ground 106, in order to achieve the stated benefit of not requiring a lead floor.

Once deployed for use as illustrated in FIGS. 1(e) and 1(f), further preparation of the transportable treatment unit 100 takes place.

The treatment unit 100 is preferably an extendable treatment unit, and once deployed for use the treatment unit may be extended.

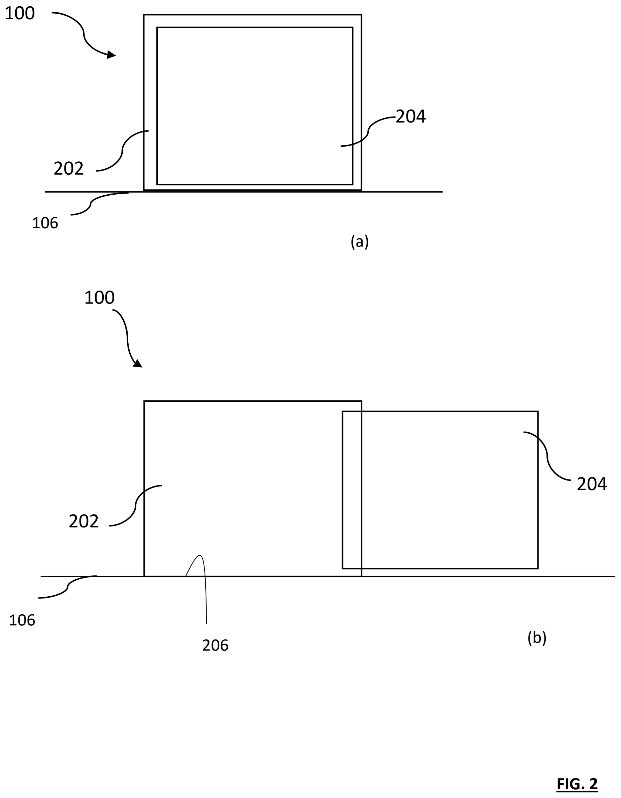

With reference to FIGS. 2(a) and 2(b) end-on views show the concept of the extendable treatment unit 100. The treatment unit 100 comprises a first portion 202 and a second portion 204. The treatment unit 100 is transformable from a first configuration in which the treatment unit 100 is in a non-expanded state (FIG. 2(a)), to a second configuration in which the treatment unit 100 is in an expanded state (FIG. 2(b)).

Preferably the transportable unit 100 is placed into its first configuration for transport, and then expanded to its second configuration after deployment on-site for use, to increase floor space.

The unit 100 is transformable between a non-expanded state and an expanded state and vice-versa, as illustrated with reference to FIGS. 2(a) and 2(b).

Although not shown in FIG. 2(b) additional supports may be provided between the ground 106 and the floor of the second portion 204, to support any space that exists after expansion.

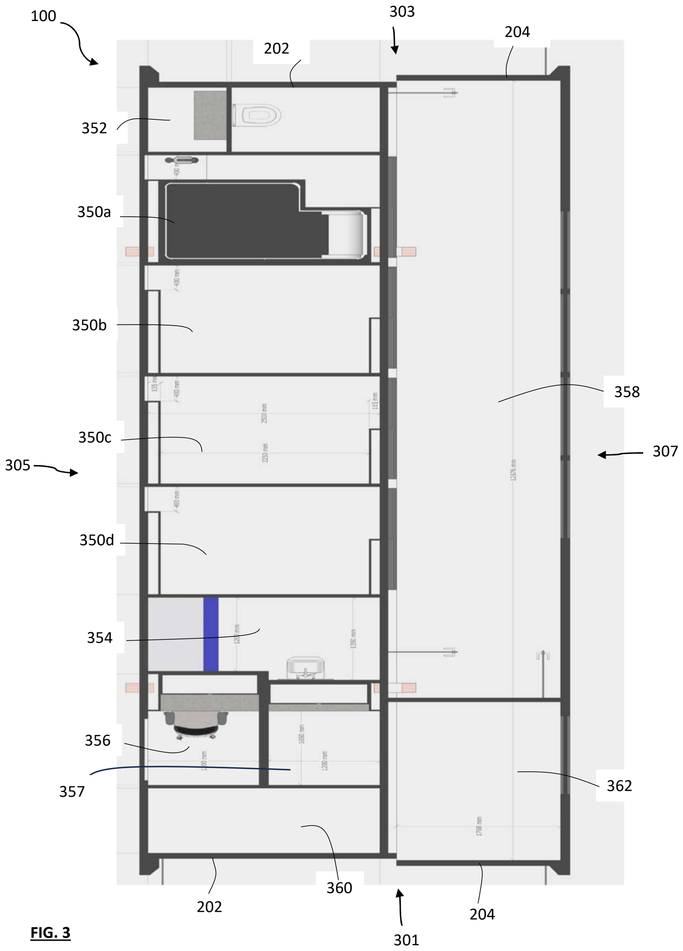

FIG. 3 is a plan view showing the layout of the extended transportable treatment unit 100. In FIG. 3, the plan view shows the transportable treatment unit 100 in its expanded state.

In the example of FIG. 3, the layout of the transportable unit 100 is configured for radioligand therapy procedures.

The transportable unit 100 has a first end side 301 and a second end side 303. The transportable unit 100 also comprises a first longitudinal side 305 and a second longitudinal side 307. The transportable unit 100 also comprises a roof and a floor, which are not shown in FIG. 3 so that the floor layout can be seen.

The treatment unit 100 includes the first portion 202 and the second portion 204. The first portion 202 is configured to remain static whilst the unit is expanded and transformed from the first configuration to the second configuration. The second portion 204 is configured to move outwardly from the first portion 202 when the unit 100 is transformed from its first configuration to its second configuration. The second portion 204 is configured to move inwardly towards the first portion 202 when the unit 100 is transformed from its second configuration to its first configuration. In FIG. 3, the unit 100 is shown in its second configuration.

The first portion 202 may include one or more treatment rooms, and preferably includes four treatment rooms 350a, 350b, 350c, 350d, each for carrying out a procedure for a single patient. A treatment room may also be referred to as a treatment module, a patient room/bay or module, uptake room/bay module, or a medical room or module.

Each treatment room 350a, 350b, 350c, 350d is a separate room. In other words, each treatment room 350a, 350b, 350c, 350d is closed off from any other areas by one or more walls, doors, windows, and has a floor and a ceiling. Each treatment room 350a, 350b, 350c, 350d may be considered a self-contained space.

It is to be noted that the treatment rooms 350a, 350b, 350c, 350d for carrying out the radioligand therapy medical procedure are located in the first portion 202 of the unit 100, i.e. they are located in the portion that is configured to remain static when the unit 100 is being transformed between its non-expanded state to its expanded state. Therefore no, or minimal, adjustments are required in treatment rooms 350a, 350b, 350c, 350d when the unit 100 is transformed between its first and second configurations.

Moreover, the first portion 202 comprises a floor 206 which is arranged to remain fixed in place as the unit 100 is transformed from the first configuration to the second configuration, and vice versa. The floor is preferably arranged to remain permanently fixed when deployed for use. The floor may remain permanently fixed through the weight of the first portion when deployer for use. The floor of the first portion remains fixed while the unit 100 is transformed from its first configuration to its second configuration. The floor 206 is also configured to remain fixed while the unit 100 is transformed from its second configuration to its first configuration.

This means that joints, articulated joints, hinges, gaps etc are not required in the floor of the first portion. This has a benefit from a hygiene point of view since such gaps, joints and hinges can become an inlet for dirt or contaminants from the outside atmosphere. In some examples, the floor of the first portion is sealed from the exterior.

Advantageously, all the fittings required for safety, including lead walls, lead glass, lead doors and lead ceilings, are provided in the first portion 202, which is static. There is no requirement to adapt the second portion 204, which is required to move, to move this heavy equipment or heavy fittings.

Each treatment room of the first portion 202 is a lead encapsulated treatment room, comprising lead walls, lead doors, lead windows and lead ceilings.

The first portion is not, however, provided with a lead floor, thus saving weight. As the floor 206 is in contact with the ground, no lead floor is required.

Each join of the lead panels (lead walls, lead doors, lead windows and lead ceilings) is sealed with an appropriate ceiling element to maintain the encapsulation of the treatment room. The first portion 202 may include a toilet room 352. A toilet in the toilet room may be configured to separate radioligand therapy waste. The toilet room may be equipped with a specialist toilet (commonly referred to as a hot toilet) which is reserved for use by patients who have been administered with radioactive isotopes, to ensure that radioactive human waste is kept separate and disposed of securely.

More specifically this toilet may be a a dedicated radioligand therapy patient toilet, and may convert aqueous radioactive waste into solid radioactive waste. Such a conversion may result in less than 1% of the radioactive waste created going into the environmental via waste water systems, with 99% being captured and converted into solid radioactive waste. The solid radioactive waste may then be stored until decayed and a radioactive reading is at background radiation levels, then disposed/incinerated of via normal clinical waste.

The first portion 202 may include a hot laboratory, dispensing room which is used to safely and securely prepare, handle, dispense and store the ligand and radioactive isotopes. One or more of these rooms may also be encapsulated rooms, as per the treatment rooms.

The first portion 202 may include an observation room 356 and a staff wellbeing area 357.

The first portion 202 may comprise a decontamination area within room 354 for decontaminating medical equipment. The decontamination room 354 may comprise a sink and shower.

The first portion 100 may comprise a room 360. The room 360 may be referred to as the unit technical hardware room which may contain IT hardware and communication systems.

The second portion 204 may be arranged for non-clinical activities. For example, the second portion 204 may comprise the reception area 362 for receiving patients and their carers. To this end a reception desk may be provided. In some examples the reception desk is movable, but can be locked in place as and when required. A waiting area may also be provided in the second portion 204.

The second portion 204 may have one or more movable elements, for facilitating movement of the second portion 204 towards and away from the first portion 202. For example, the one or more movable elements may comprise a movable floor. In some examples, the movable floor is hinged and can be folded up against longitudinal side 303. In some examples, the movable elements also comprise one or more movable partitions. The partitions may also be folded away for enabling the unit to be expanded / contracted.

The unit 100 may comprise a power connector for connecting the transportable medical unit 100 to a power source; a waste water connector for taking waste water away from the transportable medical unit 100; and a fresh water connector for connecting to a fresh water supply. In some examples, a single power connector, a single waste water connector and a single fresh water connector are provided. Therefore, in some examples, only three connections are required to fully connect the unit 100 to all necessary utilities. In some examples, the power connector, the waste water connector and the fresh water connector are located adjacent each other on a connector unit or board located on an exterior of the transportable medical unit 100. This makes connection of the unit 100 to utilities extremely quick once the unit 100 is on-site.

In some examples, a network port is also provided for connecting to a network cable. The unit 100 may additionally or alternatively be configured for mobile internet connection, such as high speed 4G and/or 5G.

The treatment unit 100 may comprise one or more external doors for enabling access into the unit 100. For example, external doors may enable access in to the second portion 204. A number of internal doors are also provided within the unit 100.

A typical movement of a patient is as follows. A patient enters second portion 204 of the unit 100, where they are greeted in reception room 362. The patient may need to wait for a period in second portion 204, before being transferred to one of rooms 350a to 350d.

A compact arrangement of a transportable medical unit is provided. For example, the entire patient flow for an radioligand therapy procedure, as well as the needs of staff and guests/carers, are catered for within a single expandable unit 100. There is no need for patients or staff to move between different units when transferring from one part of the patient flow to another. This contrasts with known mobile medical services, which will typically comprise a number of separate units on site.

It has not previously been known to be able to offer radioligand therapy service, from patient arrival to patient departure, within a single transportable unit, and that meets accreditation requirements, and particularly one which allows simultaneous treatment of multiple patients independently.

The location of the various rooms relative to each other in unit 100, as shown in FIG. 3, is not accidental or random. The positioning has been carefully considered to facilitate movement of persons and equipment in the most efficient and safest way possible (for example with regard to hygiene and radiation protection requirements). The relative positioning of rooms also enables a compact overall arrangement.

The transportable medical unit 100 can be deployed on-site very quickly, including connection to utilities.

The foregoing has described examples with reference to radioligand therapy. The examples apply equally to molecular radiotherapy.

It will be understood that the foregoing description is provided by way of example only and is non-limiting on the scope of the disclosure as defined by the appended claims.

## Claims

1. A transportable medical unit for the provision of radioligand therapy or molecular radiotherapy services, comprising:
one or more treatment rooms, each treatment room having lead walls and a lead ceiling,
wherein in a first state, the unit is lifted off the ground, and in a second state the unit is placed on the ground such that the floor of the unit is in contact with the ground.

2. The transportable medical unit of claim 1, wherein in the first state, the unit is transported and/or wherein in the second state, the unit is deployed for use.

3. The transportable medical unit of claim 1 or claim 2 wherein the medical unit is further configured to be transformable from a first configuration in which the medical unit is in a non-expanded state and a second configuration in which the medical unit is in an expanded state, the transportable medical unit comprising: a first portion which is configured to remain static whilst the medical unit is transformed from the first configuration to the second configuration, and a second portion which is configured to move outwardly when the medical unit is transformed from its first configuration to its second configuration.

4. The transportable medical unit of any preceding claim, further comprising one or more treatment rooms, preferably four treatment rooms, wherein preferably each of the one or more treatment rooms is configured for radioligand therapy or molecular radiotherapy treatment, and comprises a lead encapsulated treatment room, wherein preferably a lead encapsulated treatment room includes one or more of lead lined walls, doors, ceilings and windows, to reduce and contain radioactive exposure to patients, staff and general public from radioligand therapy or molecular radiotherapy treatments/administrations..

5. The transportable medical unit of claim 4 wherein each of the one or more treatment rooms is configured for radioligand therapy treatment, and includes a lead ceiling, doors, windows and walls.

6. The transportable medical unit of claim 4 or claim 5 wherein any join between panels forming any one of the lead lined walls, doors, ceilings and windows is sealed with sealing components.

7. The transportable medical unit of any one of claim 4 to 6, wherein the lead encapsulated treatment room does not comprise a lead floor, and in the second state the location of the floor of the medical unit on the ground provides a radioligand therapy or molecular radiotherapy sealing that a lead floor would otherwise provide.

8. The transportable medical unit of any one of claims 4 to 7 when dependent on claim 4, wherein the one or more treatment units are provided in the first portion.

9. The transportable medical unit of any one of claims 4 to 8, wherein any adaptations or modifications associated with radioligand therapy or molecular radiotherapy treatment, including lead wall where provided, are provided in the first portion.

10. The transportable medical unit of any one of claims 3 to 9 wherein the second portion provides a corridor to connect the rooms of the first portion.

11. The transportable medical unit of any one of claims 3 to 10 wherein the floor of the first portion is sealed to prevent ingress of contaminants into the first portion.

12. The transportable medical unit of any preceding claim further comprising hydraulic legs.

13. The transportable medical unit of claim 12 wherein in the first state the hydraulic legs are retracted at least partially into the transportable medical unit, and optionally in the second state the hydraulic legs are extended from the transportable medical unit to engage with the ground.

14. The transportable medical unit of any preceding claim further comprising at least one of wheels, and/or a hot lab and/or an observation room.

15. The transportable medical unit of any preceding claim further comprising a toilet, wherein the toilet is preferably configured to separately handle radioactive human waste, and wherein the toilet is particularly preferably configured to convert aqueous radioactive waste into solid radioactive waste, wherein preferably less than 1% of the radioactive waste created goes into the environmental via waste water systems, with 99% being captured and converted into solid radioactive waste, and wherein preferably the solid radioactive waste is stored until decayed and a reading is at background radiation levels, then disposed/incinerated of via normal clinical waste.
